# EUROPÄISCHE PATENTSCHRIFT

(11) **EP 1 852 093 B1**
(45) Veröffentlichungstag und Bekanntmachung des Hinweises auf die Patenterteilung: **10.09.2014**
(21) Anmeldenummer: 07008374.6
(22) Anmeldetag: 25.04.2007
(51) Int. Cl.: A61F 2/60, A61F 2/76

(54) **Verfahren zur Ermittlung der Einbauhöhe einer Prothese**
Method for determining the insertion height of a prosthesis
Procédé destiné à la détermination de la hauteur de montage d'une prothèse

(30) Priorität: 03.05.2006 DE 102006021064
(43) Veröffentlichungstag der Anmeldung: 07.11.2007
(73) Patentinhaber: Otto Bock Healthcare GmbH, 37115 Duderstadt (DE)
(72) Erfinder: Hiemisch, Christian, 37115 Duderstadt (DE)
(74) Vertreter: Lins, Edgar

(56) Entgegenhaltungen:
- DE-C- 742 944
- US-A- 3 659 294
- US-A- 4 969 911
- US-A- 5 529 576
- US-B1- 6 241 775
- DR. NÄDER M, NÄDER H G: "Otto Bock Prothesen-Kompendium - Prothesen für die untere Extremität 3. überarb. und erweiterte Auflage", 2000, SCHIELE & SCHÖN, BERLIN; DE, XP055006790, ISBN: 3-7949-0665-9 * page 13, left-hand column, paragraph 3 - right-hand column, paragraph 1 * * page 16, left-hand column, paragraph 4 - page 17, left-hand column, paragraph 1; figure 36 * * page 61, right-hand column, paragraph 2 - page 62, left-hand column, paragraph 1 * * page 65; figure 232 *

## Beschreibung

Die Erfindung betrifft ein Verfahren zur Ermittlung der Einbauhöhe einer aus mehreren Komponenten zusammengesetzten Prothese, wobei die Komponenten zumindest teilweise über Adapter und zumindest angenähert kugelförmige Flächen zueinander justierbar verbindbar sind.

Es hat sich in der Praxis durchgesetzt, Prothesen modular aufzubauen, um so Prothesen entsprechend den Bedürfnissen des jeweiligen Patienten zusammenzustellen. Für eine Unterschenkelprothese wird somit ein Schaft benötigt, der mittels eines Adapters justierbar an ein Unterschenkelrohr ansetzbar ist, das wiederum justierbar mit einem Fußmodul verbunden wird. Das Fußmodul kann dabei entsprechend den Bedürfnissen des Patienten für einen hohen Gehkomfort, sportliche Belastungen usw. speziell ausgelegt sein. Beispiele für Prothesen mit einem Justieradapter sind beispielsweise in US 3,659,294, US 4,969,911, US 5,529,576 und "Otto Bock Prothesen-Kompendium - Prothese für die untere Extremität", 3. überarb. Und erweiterte Auflage, Berlin, herausgegeben von Max Näder und Hans-Georg Näder, beschrieben.

Für einen am Oberschenkel amputierten Patienten kommt der Einsatz eines Kniegelenks hinzu, das auf der Höhe des natürlichen Knies des gesunden Beins angeordnet sein muss.

Die Ermittlung der Einbauhöhe einer derartig zusammengesetzten Prothese erfolgt durch Vermessung der Prothese und Anpassung eines ggf. längenverstellbaren Einzelteils, wie beispielsweise eines längenverstellbaren Unterschenkelteil-Rohrteils. Es zeigt sich, dass die Vermessung der Prothese und die Bestimmung der für den Patienten benötigen Bauhöhe einen erfahrenen Orthopädiemechaniker voraussetzt und auch dann häufig ungenau erfolgt, obwohl die Bestimmung der benötigen Bauhöhe aus den Patientendaten in ausreichender Genauigkeit ermittelbar ist.

Die DE 742 944 beschreibt eine Messvorrichtung, in die ein Probekunstbein, mit dem der Patient eine längere Zeit einwandfrei gelaufen ist, einspannbar ist, wobei zahlreiche Messleeren verschiebbar angeordnet sind. Durch Einstellung bestimmter Punkte ergeben sich somit mit den Messleeren ablesbare Einstellwerte, die es ermöglichen, eine zu dem Probekunstbein identische Prothese herzustellen. Die Ermittlung einer Gesamt-Bauhöhe einer modular aufgebauten Prothese aus Abmessungen der Prothesenteile ist hier weder angesprochen noch möglich.

US 6,241,775 B1 beschreibt ein digitales Werkzeug zur Zusammenstellung von modularen Prothesen, wobei die Prothesenkomponenten jeweils in einem Datensatz abgebildet werden. Die Prothesenkomponenten einer Klasse (beispielsweise Fußprothesen) müssen mit Prothesenkomponenten anderer Klassen (beispielsweise Knöchelkomponenten, Unterschenkelkomponenten, Kniegelenkkomponenten usw.) zu einer vollständigen Prothese zusammengestellt werden. Die Datensätze enthalten dabei Hinweise, welche Knöchelkomponenten mit welchen Fußprothesen und mit welchen Unterschenkelteilen kombinierbar ist, ggf. unter Einschaltung eines Adapters. Die Datensätze enthalten somit die Informationen über die zusammenstellbaren Komponenten bzw. über die Unverträglichkeit bestimmter Komponenten miteinander. Die Ermittlung der Aufbauhöhe einer Modularprothese ist dabei nicht angesprochen.

Eine ähnliche Tabelle über miteinander zu einer Prothese zusammenstellbaren Prothesenkomponente findet sich in dem "Prothesenkompendium" in Abbildung 232 auf Seite 65. Daraus wird beispielsweise deutlich, welche Prothesenteile welcher Fußprothese aus einer Auswahl von sieben Fußprothesen kombinierbar sind, um beispielsweise den Anschluss zu einem Unterschenkelteil und einem Kniegelenk herzustellen. Für die Ermittlung der Aufbauhöhe einer derartigen Prothese enthält auch das "Prothesen-Kompendium" keine Hinweise.

Der vorliegenden Erfindung liegt daher die Aufgabe zugrunde, die Ermittlung der Einbauhöhe einer aus mehreren Komponenten zusammengesetzten Prothese zu erleichtern und fehlerfreier zu gestalten.

Die Lösung dieser Aufgabe gelingt mit einem Verfahren der eingangs erwähnten Art erfindungsgemäß dadurch, dass jede Komponente eine Systemhöhenangabe zugeordnet wird, wobei für diejenigen Komponenten, die mittels einer zumindest angenähert kugelförmigen Fläche justierbar sind, die Systemhöhenangabe ein bis zum Mittelpunkt der jeweiligen kugelförmigen Fläche reichendes Maß beinhaltet, und dass diese Systemhöhenangaben für die aneinander gesetzten Komponenten zur Ermittlung der Bauhöhe addiert werden.

Der Erfindung liegt die Erkenntnis zugrunde, dass die Ermittlung der effektiven Einbauhöhen der Komponenten innerhalb der Prothese problematisch ist und zu den fehlerhaften Messungen Anlass gegeben hat. Demgemäß wird jeder Komponente, die zum Aufbau einer Prothese verbaubar ist, eine Systemhöhenangabe zugeordnet, die für diejenigen Komponenten, die über eine zumindest angenähert kugelförmige Fläche justierbar ausgebildet sind, ein virtuelles Maß enthält, nämlich ein Maß, das bis zum Mittelpunkt der jeweiligen kugelförmigen Fläche reicht. Durch die Verwendung dieser Systemhöhenangaben kann eine Addition für die Komponenten vorgenommen werden, weil die über eine kugelförmige Fläche miteinander justierbar angeordneten Komponenten einen übereinstimmenden Mittelpunkt der jeweiligen Kugelflächen aufweisen. Die Systemhöhenangabe kann somit bei der Fertigung der Kom-ponente diesem zugeordnet werden, sodass lediglich eine Addition der Systemhöhenangaben erforderlich ist um die effektive Einbauhöhe der Prothese zu ermitteln. Zu beachten ist dabei, dass eine Komponente dabei eine negative Systemhöhenangabe aufweisen kann, beispielsweise eine konkav ausgebildete Schaftaufnahme, bei der der Kugelmittelpunkt innerhalb des Schafts bzw. des Amputationsstumpfs liegt. In diesem Fall muss der negative Wert der Systemhöhenangabe addiert werden, um zur effektiven Einbauhöhe der Prothese zu gelangen.

Selbstverständlich ist es bei dem erfindungsgemäßen Verfahren möglich, die Systemhöhe der Prothese auch bis zu einer in zweckmäßigerweise ausgewählten Referenzlinie zu bestimmen, beispielsweise bis zur Kniespalthöhe des gesunden Beins, um sicherzustellen, dass die bis zum Oberschenkel ragende Prothese ein Kniegelenk aufweist, das in korrekter Höhe relativ zu dem gesunden Bein des Patienten angeordnet ist, um so zu einem möglichst natürlichen Gangverhalten des Patienten zu gelangen.

Eine modulare Prothese weist im Allgemeinen wenigstens eine längenveränderliche Komponente auf, die mit einer ablesbaren Längeneinstellung versehen ist. In diesem Fall ist es zweckmäßig, wenn die jeweilige ablesbare Längeneinstellung direkt proportional in die Systemhöhenangabe einfließt, um so die zutreffende Systemhöhenangabe der längenveränderlichen Komponente zu bestimmen.

Die Erfindung soll im Folgenden anhand eines in der Zeichnung dargestellten Ausführungsbeispiels näher erläutert werden. Es zeigen:
- Figur 1: Komponenten einer Unterschenkel-Prothese;
- Figur 2: die Komponenten der Unterschenkel-Prothese gemäß Figur 1 mit Systemhöhenangaben;
- Figur 3: eine zusammengesetzte Prothese;
- Figur 4: eine schematische Darstellung der Situation an einer Oberschenkekl-Prothese.

Die in der Zeichnung dargestellte Prothese besteht aus drei Komponenten, nämlich einem Fußmodul 1, einem Unterschenkelmodul 2 und einer Schaftaufnahme 3.

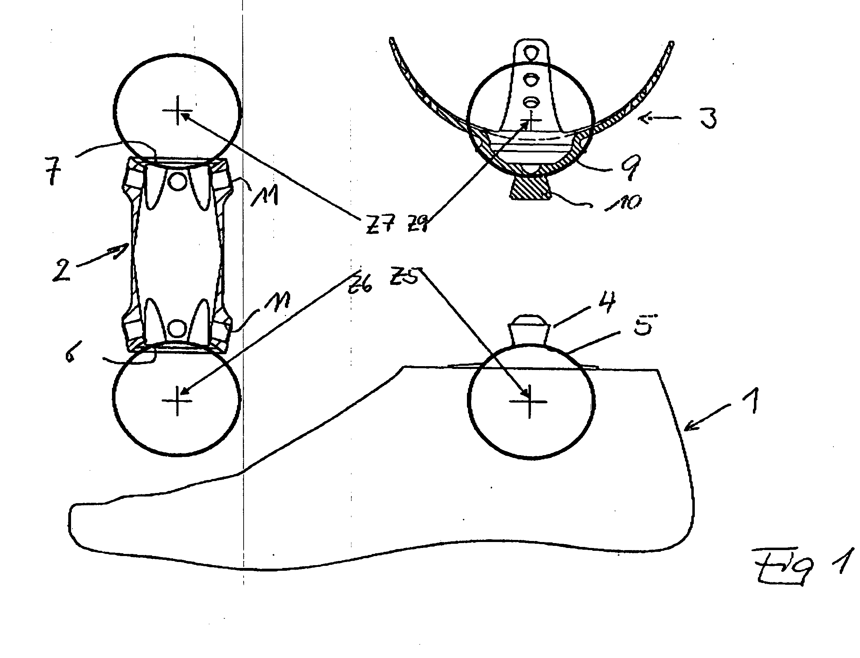

Figur 1 verdeutlicht, dass das Fußmodul 1 mit einem Adapter 4 versehen ist, der einen kugelförmige Justierfläche 5 aufweist.

Mittels des Adapters 4 ist das Unterschenkelmodul 2 mit dem Fußmodul 1 verbindbar, wobei das Unterschenkelmodul 2 am unteren Ende eine mit der kugelförmigen Justierfläche 5 korrespondierende konkave kugelförmige Justierfläche 6 aufweist.

Am oberen Ende weist das Unterschenkelmodul 2 eine entsprechende konkave kugelförmige Justierfläche 7 auf, die den gleichen Kugelradius aufweist wie eine kugelförmige (konvexe) Justierfläche 9 eines Adapters 10 der Schaftaufnahme 3.

Die Adapter 4, 10 weisen in an sich bekannter Weise die Form eines umgekehrten Pyramidenstumpfes mit Justierflächen auf, die mit (nicht dargestellten) Justierschrauben zusammenwirken, die in Gewindebohrungen 11 der Adaptergegenstücke an den Enden der Unterschenkel-Prothese 2 zusammenwirken.

Die kugelförmigen Justierflächen 5, 6, 7, 9 weisen jeweils zugehörige Kugelmittelpunkte Z5,, Z6, Z7, Z9 auf.

Die vorliegende Erfindung beruht auf der Erkenntnis, dass bei einem korrekten Zusammenbau der Prothesenkomponenten 1, 2, 3 die Kugelmittelpunkte Z5 und Z6 einerseits und Z7 und Z9 andererseits zusammenfallen.

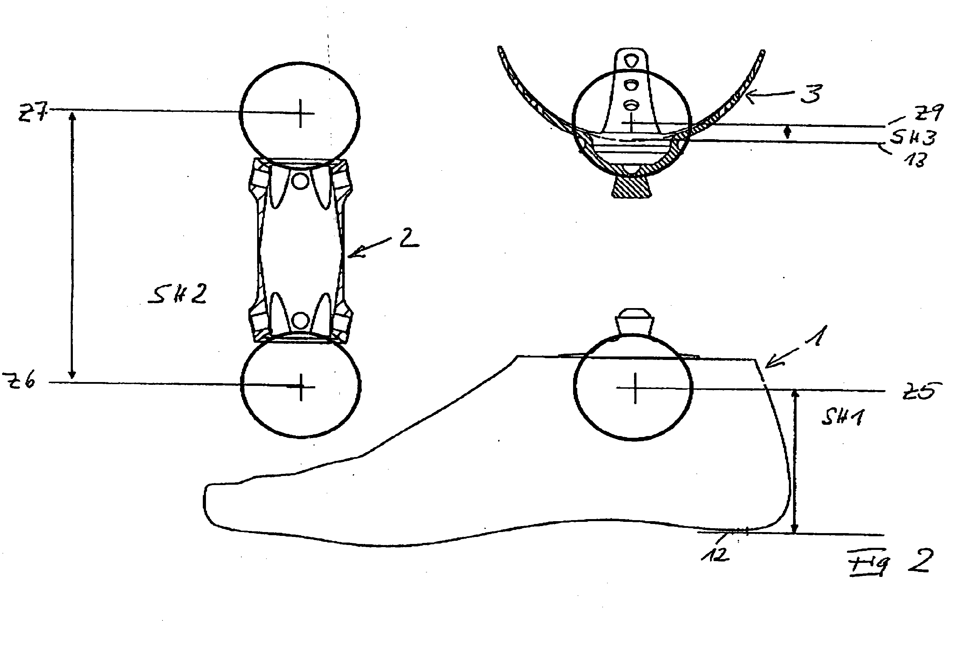

Demgemäß sind gemäß Figur 2 den Komponenten 1, 2, 3 Systemhöhenangaben SH1, SH2 und SH3 zugeordnet.

Die Systemhöhenangabe SH1 des Fußmoduls 1 ergibt sich aus dem Abstand zwischen einer Auflagefläche 12 des Fußmoduls 1 und der Höhe des Kugelmittelpunkts 25.

In einem Ausführungsbeispiel beträgt die Systemhöhe SH1 = 72 mm.

Die Systemhöhenangabe SH2 für das Unterschenkelmodul 2 ergibt sich aus dem Abstand der Kugelmittelpunkte Z6 und Z7. In einem Ausführungsbeispiel beträgt die Systemhöhe SH2 = 112 mm.

Die Systemhöhe SH3 ergibt sich aus dem Abstand des Kugelmittelpunkts Z9 von einer Schaftendenhöhe 13 eines von der Schaftaufnahme 3 aufgenommenen (nicht dargestellten) Schafts für einen Amputationsstumpf.

Da der Kugelmittelpunkt Z9 oberhalb der Schaftendenhöhe 13 liegt, ist die Angabe für die Systemhöhe SH3 negativ. In einem Ausführungsbeispiel beträgt die Systemhöhe SH3 = -5mm.

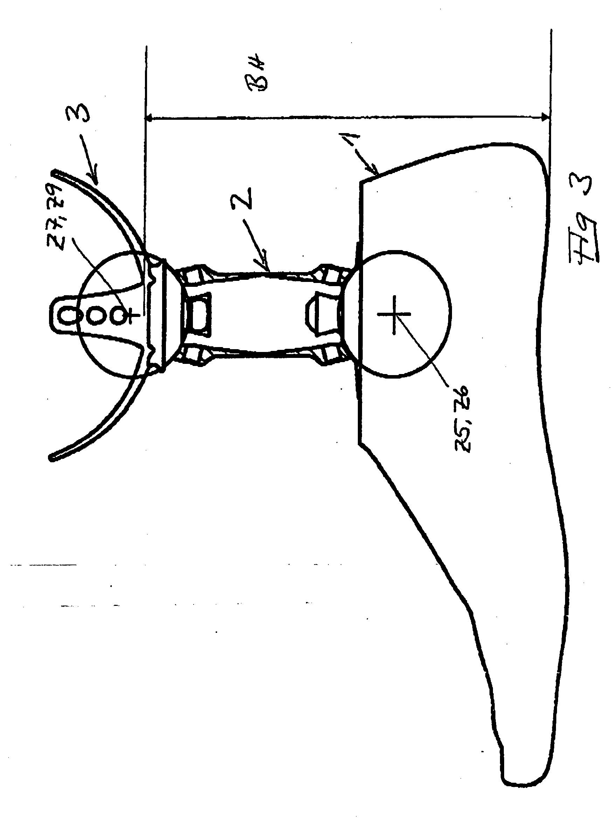

Figur 3 verdeutlicht die aus den Komponenten 1, 2, 3 zusammengesetzte Prothese. Die Kugelmittelpunkte Z5 und Z6 einerseits und Z7 und Z9 andererseits fallen zusammen, wenn die Prothese exakt montiert ist. Eine relevante Einbauhöhe BH der Prothese ergibt sich aus der Addition der Systemhöhen SH1 + SH2 + SH3. In dem angegebenen Ausführungsbeispiel ergibt sich die Einbauhöhe BH somit zu 72 + 112 - 5 mm = 179 mm.

Es ist somit ohne weiteres erkennbar, dass die relevante Einbauhöhe BH in einfacher Weise durch die den Komponenten 1, 2, 3 zugeordneten Systemhöhen SH1, SH2 und SH3 ermittelbar ist.

Für den Fall, dass eine Komponente längenveränderlich ausgebildet ist, also ein teleskopisch verschiebbares Rohrstück aufweist, wird die Einstellung auf dem Rohrstück ablesbar gestaltet. Der Komponente wird eine Standard-Systemhöhe zugeordnet, die durch die ablesbare Längeneinstellung durch Addition bzw. Subtraktion eines abgelesenen Abweichungswerts von einer Ausgangsstellung modifiziert, woraus sich der nach der Längeneinstellung relevante Wert für die Systemhöhe SH ergibt.

In dem dargestellten Ausführungsbeispiel ist eine Unterschenkel-Prothese für einen unterhalb des Kniegelenks amputierten Patienten zugrunde gelegt worden. Wird eine Prothese für einen oberhalb des Kniegelenks, also am Oberschenkelknochen amputierten Patienten aufgebaut, ergibt sich das Problem der Bestimmung der Einbauhöhe BH bis zur Schaftendenhöhe, zusätzlich aber die weitere Bedingung, dass das in diese Prothese eingesetzte Kniegelenk auch auf einer zutreffenden Höhe angeordnet ist.

Wie Figur 3 verdeutlicht, wird hierzu am gesunden Bein des Patienten die Kniespalthöhe KSd zwischen dem Kniespalt des gesunden Beins und der Auflagefläche 12 als Referenzhöhe bestimmt. Die gesamte Einbauhöhe BH ergibt sich somit als Summe der Kniespalthöhe KSd und eines Abstandes KSp zwischen dem Kniespalt und einer Schaftendenhöhe 14 eines den Oberschenkel-Amputationsstumpf aufnehmenden (hier nicht dargestellten) Schafts.

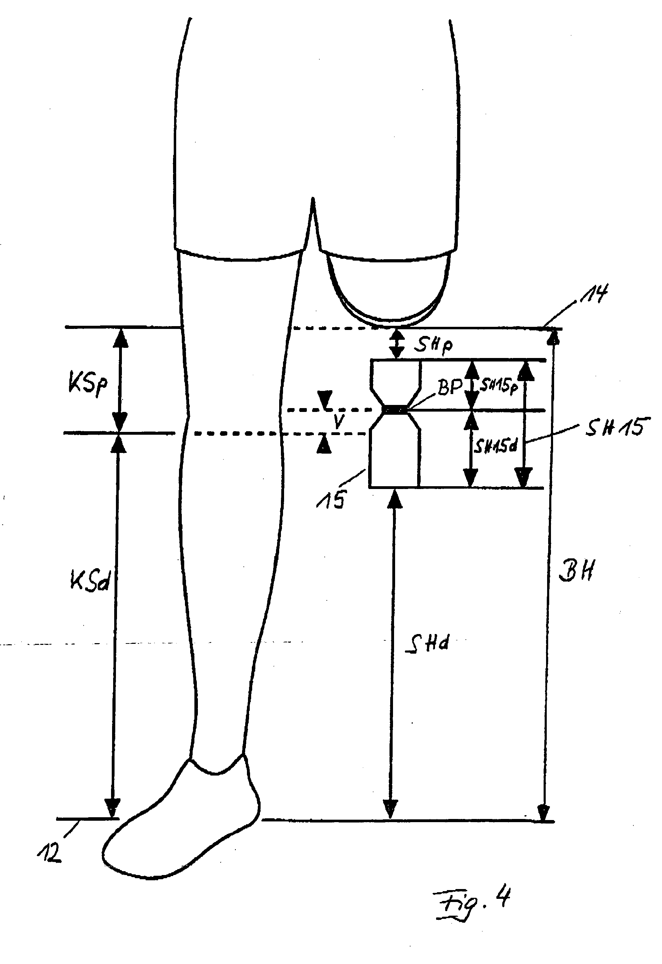

In Figur 4 ist ferner schematisch ein Kniegelenk 15 dargestellt, für das ein Aufbau-Bezugspunkt BP bestimmt ist. Für das Kniegelenk 15 wird ebenfalls eine Systemhöhe SH15 in analoger Weise wie für Komponenten 1, 2, 3 bestimmt. Die Systemhöhe SH15 des Kniegelenks 15 wird unterteilt in eine proximale Systemhöhe SH15p und in eine distale Systemhöhe SH15d, die in der Höhe des Aufbau-Bezugspunkt BP aneinander anschließen.

Bei einem monozentrischen Kniegelenk, das mit einer ortsfesten Drehachse aufgebaut ist, ergibt sich der Aufbau-Bezugspunkt BP aus der Höhe der Drehachse. Bei einem polyzentrischen Kniegelenk, beispielsweise bei einer Vier-Achs-Anordnung wird der Aufbau-Bezugspunkt durch das obere vordere Achsgelenk bestimmt. Es ist zweckmäßig, den Aufbau-Bezugspunkt BP mit einem Versatz V oberhalb der Kniespalthöhe KSd anzuordnen, beispielsweise etwa 20 mm oberhalb der Kniespalthöhe KSd.

Für das Kniegelenk 15 wird somit eine Gesamt-Systemhöhe SH15 ermittelt, durch deren Aufbau-Bezugspunkt BP in Relation zu der Kniespalthöhe KSd des gesunden Beins eine verfügbare proximale Systemhöhe SHp und eine verfügbare distale Systemhöhe SHd bestimmt werden. Diese verfügbaren Systemhöhen SHp und SHd können dann mit den Systemhöhen der für den Aufbau der Oberschenkel-Prothese vorhandenen Komponente, im Allgemeinen unter Verwendung wenigstens einer längenverstellbaren Komponente, ausgefüllt werden. Selbstverständlich wird bei der Ermittlung der Systemhöhe SH15 des Kniegelenks ebenfalls berücksichtigt, ob das Kniegelenk 15 an seiner Oberseite und Unterseite mit einer kugelförmigen Justierfläche abgeschlossen ist, wodurch sich das ggf. virtuelle Maß SH15 für die Systemhöhe - wie anhand der Figuren 1 bis 3 erläutert worden ist - ergibt.

## Patentansprüche

1. Verfahren zur Ermittlung der Einbauhöhe (BH) einer aus mehreren Komponenten (1, 2, 3, 15) zusammengesetzten Prothese, wobei die Komponenten (1, 2, 3, 15) zumindest teilweise über Adapter (4, 10) und zumindest angenähert kugelförmigen Flächen (5, 6, 7, 9) zueinander justierbar verbindbar sind, **dadurch gekennzeichnet, dass** jeder Komponente eine Systemhöhenangabe (SH1, SH2, SH3, SH15) zugeordnet wird, die für mittels einer zumindest angenähert kugelförmlgen Fläche (5, 6, 7, 9) justierbare Komponenten (1, 2, 3, 15) ein bis zum Mittelpunkt (Z5, Z6, Z7, Z9) der jeweiligen kugelförmigen Fläche (5, 6, 7, 9) reichendes Maß beinhaltet und dass diese Systemhöhenangaben (SH1, SH2, SH3, SH15) für die aneinander gesetzten Komponenten (1, 2, 3, 15) zur Ermittlung der Einbauhöhe (BH) addiert werden.

2. Verfahren nach Anspruch 1, **dadurch gekennzeichnet, dass** einem längenveränderlichen Einzelteil eine zur jeweiligen Längeneinstellung direkt proportionale Systemhöhenangabe zugeordnet wird.

3. Verfahren nach Anspruch 1 oder 2, **dadurch gekennzeichnet, dass** für wenigstens eine Komponente (15) die ermittelte Systemhöhe (SH15) unter Bezugnahme auf einen Einbau-Bezugspunkt in Teil-Systemhöhen (SH15p, SH15d) unerteilt wird und dass der Einbau-Bezugspunkt (BP) an der Höhe (KSd) eines Bezugspunkts eines gesunden Gliedes orientiert wird.

4. Verfahren nach Anspruch 3, **dadurch gekennzeichnet, dass** der Bezugspunkt des gesunden Gliedes der Kniespalt des Kniegelenks ist.

5. Verfahren nach Anspruch 4, **dadurch gekennzeichnet, dass** der Einbau-Bezugspunkt (BP) eines künstlichen Kniegelenks (15) um einen vorgegebenen Versatz (V) oberhalb der Höhe (KSd) des Kniegelenkspalts des gesunden Gliedes angeordnet ist.

## Claims

1. Method for determining the overall height (BH) of a prosthesis composed of several components (1, 2, 3, 15), in which at least some of the components (1, 2, 3, 15) can be connected adjustably to one another via adapters (4, 10) and at least approximately spherical surfaces (5, 6, 7, 9), **characterized in that** each component is assigned a system height value (SH1, SH2, SH3, SH15) which, for components (1, 2, 3, 15) that are adjustable by means of an at least approximately spherical surface (5, 6, 7, 9), includes a dimension reaching as far as the midpoint (Z5, Z6, Z7, Z9) of the respective spherical surface (5, 6, 7, 9), and **in that** these system height values (SH1, SH2, SH3, SH15) for the components (1, 2, 3, 15) mounted on one another are added together to determine the overall height (BH).

2. Method according to Claim 1, **characterized in that** an individual part of adjustable length is assigned a system height value that is directly proportional to the respective length setting.

3. Method according to Claim 1 or 2, **characterized in that**, for at least one component (15), the determined system height (SH15) is subdivided into subsidiary system heights (SH15p, SH15d) with respect to a reference point, and **in that** the reference point (BP) is oriented with respect to the height (KSd) of a reference point of an intact limb.

4. Method according to Claim 3, **characterized in that** the reference point of the intact limb is the knee gap of the knee joint.

5. Method according to Claim 4, **characterized in that** the reference point (BP) of an artificial knee joint (15) is arranged at a predetermined offset (V) above the height (KSd) of the knee gap of the intact limb.

## Revendications

1. Procédé pour déterminer la hauteur de montage (BH) d'une prothèse composée de plusieurs composants (1, 2, 3, 15), dans lequel les composants (1, 2, 3, 15) sont susceptibles d'être relié les uns aux autres de façon à pouvoir être ajustés les uns par rapport aux autres au moins partiellement via des adaptateurs (4, 10) et via des surfaces au moins approximativement de forme sphérique (5, 6, 7, 9),
**caractérisé en ce qu'**une indication de hauteur systémique (SH1, SH2, SH3, SH15) et associée à chaque composant, hauteur qui, pour des composants (1, 2, 3, 15) susceptibles d'être ajustés au moyen d'une surface au moins approximativement de forme sphérique (5, 6, 7, 9), inclut une mesure allant jusqu'au centre (Z5, Z6, Z7, Z9) de la surface de forme sphérique respective (5, 6, 7, 9), et **en ce que** ces indications de hauteur systémique (SH1, SH2, SH3, SH15) sont additionnées pour les composants posés les uns contre les autres (1, 2, 3, 15) pour déterminer la hauteur de montage (BH).

2. Procédé selon la revendication 1, **caractérisé en ce qu'**une indication de hauteur systémique directement proportionnelle au réglage de longueur respectif est associée à une pièce individuelle dont la longueur peut être variée.

3. Procédé selon la revendication 1 ou 2, **caractérisé en ce que** pour au moins un composant (15) la hauteur systémique déterminée (SH15) est subdivisée, en se référant à un point de référence de montage, en hauteurs systémiques partielles (SH15p, SH15d), et **en ce que** le point de référence de montage (PB) est orienté à la hauteur (KSd) d'un point de référence d'un membre sain.

4. Procédé selon la revendication 3, **caractérisé en ce que** le point de référence du membre sain est la face inférieure du ménisque de l'articulation du genou.

5. Procédé selon la revendication 4, **caractérisé en ce que** le point de référence de montage (BP) d'une articulation artificielle du genou (15) est agencé avec un décalage prédéterminé (V) au-dessus de la hauteur (KSd) de la face inférieure du ménisque de l'articulation du genou du membre sain.
